# EUROPEAN PATENT APPLICATION

(11) **EP 2 959 902 A1**
(43) Date of publication of application: **30.12.2015**
(21) Application number: 15176482.6
(22) Date of filing: 21.07.2010
(51) Int. Cl.: A61K 31/439, A61K 31/444, A61K 31/501, A61K 31/506, A61P 25/14

(54) **USE OF AZABICYCLOALKYL DERIVATIVES FOR THE TREATMENT OR PREVENTION OF ATAXIA**

(30) Priority: 23.07.2009 US 227951 P
(62) Divisional of application: 10734744.5
(71) Applicant: Novartis AG, 4056 Basel (CH)
(72) Inventor: FEUERBACH, Dominik, 4002 Basel (CH); GOMEZ-MANCILLA, Baltazar, 4002 Basel (CH)
(74) Representative: Appleyard Lees

(57) **Abstract**

The invention concerns the use of azabicycloalkyl derivatives for the treatment, prevention or delay of progression of ataxia.

## Description

The present invention relates to pharmaceutical uses of certain azabicycloalkyl derivatives or pyrrolidine-2-one derivatives.

Ataxia is a disorder of the central nervous system wherein the patient is unable to coordinate muscles for the execution of voluntary movement; for review see T Klockgether, 2007 (T Klockgether, Parkinsonism and Related Disorders, 13, S391-S394, 2007). Typical symptoms of ataxia are gait dysfunctions, imbalance, impaired limb coordination and altered speech. In most ataxia disorders, the ataxia is due to degeneration of the cerebellar cortex and its afferent or efferent fibre connection, typical affected brain regions are cerebellum, posterior column, pyramidal tracts and basal ganglia. Ataxia may lead to a decreased motoneuron function. Ataxia is typically classified into hereditary and non-hereditary ataxias.

Hereditary ataxias are further classified into autosomal recessive and autosomal dominant ataxias. Autosomal recessive ataxias are, for example, Friedreichs ataxia (FRDA), Ataxia telangiectasia (AT), Autosomal recessive ataxia with oculomotor apraxia type 1, Autosomal recessive ataxia with oculomotor apraxia type 2, Spinocerebellar ataxia with axonal neuropathy, Abetalipoproteinemia, Ataxia with isolated vitamin E deficiency, Refsums disease and Cerebrotendinous xanthomatosis. Autosomal dominant ataxias are, for example, Spinocerebellar ataxias (SCA), which can be further classified into ataxias associated with translated CAG repeat expansions (SCA1, 2, 3, 6, 7 and 17), ataxias associated with untranslated repeat expansions in non-coding regions (SCA8, 10 and 12), ataxias associated with point-mutations (SCA5, 13, 14 and 27). SCA3 is also known as Machado-Joseph disease.

Non-hereditary ataxias can be further classified into degenerative and acquired ataxias. Degenerative ataxias are, for example, multiple system atrophy ataxia and sporadic adult-onset ataxias. Acquired ataxias can be, for example, associated with alcoholic/toxin-caused cerebellar degeneration or paraneoplastic cerebellar degeneration.

As ataxia may result from a wide range of underlying causes - either genetic (e.g. FRDA is caused in the vast majority of patients by a homozygous intronic GAA repeat expansion of the frataxan gene, which leads to a lower expression level of frataxin, which ultimately leads to an accumulation of mitochondrial iron) or environmental (e.g. ataxia associated with toxin-caused cerebellar degeneration) - many potential therapies are discussed in the field. An overview relating to potential therapies for ataxias is given in the review from T Klockgether, 2007. For FRDA, for example, therapies based on idebenone, a short-chain coenzyme Q10 analogue, or deferiprone, a penetrant oral iron chelator, showed effects in clinical trials (T Klockgether, 2007; WO2007095728). Additionally, therapies based on porphyrine compounds comprising a complexed Ca²⁺ ZN²⁺ or Mg²⁺ ion showed beneficial effects in pre-clinical models (WO2007085036).

Recently, TA Zesiewicz published information about ataxia improvements seen for some patients in clinical trials of varenicline (7,8,9,10-tetrahydro-6,10-methano-6H-pyrazino-(2,3-h)(3)-benzazepine), a medicament to treat smoking addiction. Two patients with FRDA (TA Zesiewicz et al, J Clinical Neuromuscular Disease, 10(4), 191-193, 2009) and two patients with SCA3/SCA14 (TA Zesiewicz et al, Clinical Neuropharmacology, 31(6), 363-365, 2008), which all received varenicline p.o. at a dose of 0.5mg/kg per day, showed improvements. Although varenicline can act as a partial agonist at the alpha-4/beta-2 nicotinic acetylcholine receptor (α4β2-nAChR; Kᵢ in oocytes: 0.4nM) and as a full agonist at the α7-nAChR (Kᵢ in oocytes; 125nM), its functional behaviour changes with dosing. At low doses varenicline desensitizes the receptors; only at high doses it leads to (partial) receptor activation. At 0.5mg/kg in humans, varenicline is believed to desensitize both receptors, i.e. to be a functional antagonist (H Rollema et al, Biochemical Pharmacology, 78, 813-824, 2009),

During clincial use of varenicline, adverse effects have been reported, such as nausea, sleep disturbance, constipation, vivid dreams, flatulence and vomiting. Further, potentially due to said adverse effects, treatment of smoking addiction with varenicline is limited to maximally 24 weeks; whereas for ataxia, a life-long treatment would be indicated.

Although some benefits are seen in the above-described clinical trials/pre-clinical models, there is no effective treatment of ataxia with acceptable side-effects available to patients. Consequently, there is a need to provide drugs for the treatment of ataxia.

It has been found that a compound selected from the group consisting of
(A) a compound of formula (I) 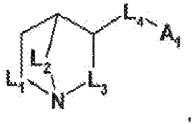 wherein
   L₁ is -CH₂-; L₂ is -CH₂- or -CH₂-CH₂-, and L₃ is -CH₂- or -CH(CH₃)- or L₁ is -CH₂-CH₂-; L₂ is -CH₂-; and L₃ is -CH₂-CH₂-;
   L₄ is a group selected from 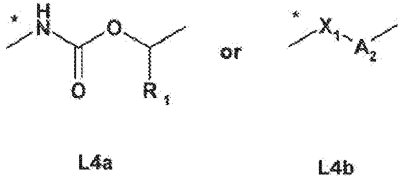 wherein the bond marked with the asterisk is attached to the azabicycloalkyl moiety;
   R₁ is hydrogen or C₁₋₄alkyl;
   X₁ is -O- or -NH-,
   A₂ is selected from 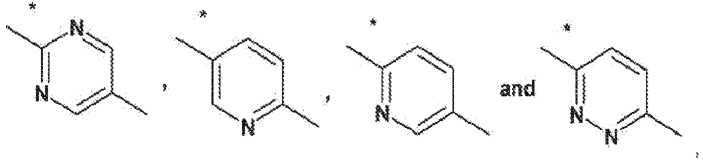 wherein the bond marked with the asterisk is attached to X₁;
   A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen;
   each R₂ independently is C₁₋₆alkyl, C₁₋₆halogenalkyl, C₁₋₆alkoxy, C₁₋₆halogenalkoxy, halogen, cyano or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C_{1- 6}halogenalkoxy, halogen or cyano, and wherein a substituent on a nitrogen in a heterocyclic ring system may not be halogen;
   or two R₂ at adjacent ring atoms form a C₃₋₄alkylene group, wherein 1-2 carbon atoms may be replaced by X₂, and wherein the C₃₋₄alkylene group may be substituted once or more than once by R₃;
   each X₂ independently is -O- or -N(R₄)-;
   each R₄ independently is hydrogen or C₁₋₆alkyl; and
   each R₃ independently is halogen or C₁₋₆alkyl; and
(B) a compound of formula (II), 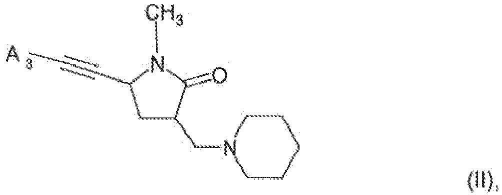 wherein
   A₃ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₅, and wherein a substituent on a nitrogen in a heterocyclic ring system may not be halogen;
   each R₅ independently is C₁₋₆alkyl, C₁₋₆hatogenalkyl, C₁₋₆alkoxy, C₁₋₆halogenatkoxy, halogen, cyano, amino or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen;
   or two R₅ at adjacent ring atoms form a C₃₋₄alkylene group, wherein 1-2 carbon atoms may be replaced by X₃, and wherein the C₃₋₄alkylene group may be substituted once or more than once by R₆;
   each X₃ independently is -O- or -N(R₇)-;
   each R₇ independently is hydrogen or C₁₋₆alkyl; and
   each R₆ independently is halogen or C₁₋₆alkyl;
   in free base form or in pharmaceutically acceptable acid addition salt form may be used in the treatment, prevention or delay of ataxia.

Accordingly, a first aspect of the invention concerns the use of a compound of formula (I) or a compound of formula (II) for the treatment (whether therapeutic or prophylactic), prevention or delay of progression of ataxia.

The compounds of formula (I) or compounds of formula (II) are α7-nAChR agonists. These compounds and their use as pharmaceuticals are described in WO2001/85727, WO2004/022556, WO2005/118535, WO2Q05/123732, WO2006/005608. WO2007/045478, WO2007/068476 and WO2007/068475. α7-nAChR are widely distributed throughout the central nervous system (CNS) and are potential targets for CNS diseases, such as neuropsychiatric diseases, e.g. cognitive dysfunction (SL Cincotta et al, Current Opinion in Investigational Drugs, 9(1), 47-56, 2008; LM Broad et al, Drugs of the Future, 32(2), 161-170, 2007). Furthermore, it was found that activation of α7-nAChR promotes survival of cultured spinal cord motoneurons (ML Messi, FEBS Letters, 411, 32-38, 1997).

A further aspect of the invention relates to a method for the treatment, prevention or delay of progression of ataxia in a subject in need of such treatment, which comprises administering to said subject a therapeutically effective amount of a compound of formula (I) or a compound of formula (II),

A further aspect of the invention relates to a pharmaceutical composition comprising a compound of formula (I) or a compound of formula (II) for the treatment, prevention or delay of progression of ataxia.

A further aspect of the invention relates to the use of a compound of formula (I) or a compound of formula (II) for the manufacture of a medicament for the treatment, prevention or delay of progression of ataxia.

A further aspect of the invention relates to a compound of formula (I) or a compound of formula (II) for the treatment, prevention or delay of progression of ataxia.

In particular, preferred compounds of the invention should be well absorbed from the gastrointestinal tract, should be sufficiently metabolically stable and possess favorable pharmacokinetic properties.

Further preferred compounds of the invention bind in-vivo potently to α7-nAChRs whilst showing little affinity for other receptors, especially for XXX, YYY and ZZZ.

Further preferred compounds of the invention cross the blood brain barrier effectively. Preferred compounds of the invention should be non-toxic and demonstrate few side-effects. Furthermore, a preferred compound of the invention will be able to exist in a physical form that is stable, non-hygroscopic and easily formulated.

Unless indicated otherwise, the expressions used in this invention have the following meaning:
"Alkyl" represents a straight-chain or branched-chain alkyl group, for example, methyl, ethyl, n- or iso-propyl, n-, iso-, sec- or tert-butyl, n-pentyl, n-hexyl; C₁₋₆alkyl preferably represents a straight-chain or branched-chain C₁₋₄alkyl with particular preference given to methyl, ethyl, n-propyl, iso-propyl and tert-butyl.

Each alkyl part of "alkoxy", "halogenalkyl" and so on shall have the same meaning as described in the above-mentioned definition of "alkyl", especially regarding linearity and preferential size.

A substituent being substituted "once or more than once", for example as defined for A₁, is preferably substituted by one to three substituents,

Halogen is generally fluorine, chlorine, bromine or iodine; preferably fluorine, chlorine or bromine. Halogenalkyl groups preferably have a chain length of 1 to 4 carbon atoms and are, for example, fluoromethyl, difluoromethyl, trifluoromethyl, chloromethyl, dichloromethyl, trichloromethyl, 2,2-,2-trifluoroethyl, 2-fluoroethyl, 2-chloroethyl, pentafluoroethyl, 1,1-difluoro-2,2,2-trichloroethyl, 2,2,2-trichloroethyl, 1,1,2,2-tetrafluoroethyl, 2,2,3,3-tetrafluoropropyl, 2,2,3,3,3-pentafluoropropyl or 2,2,3.4,4,4-hexafluorobutyl; preferably -CF₃, -CHF₂, -CH₂F₃-CHF-CH₃, -CF₂CH₃, or -CH₂CF₃.

In the context of the invention, the definitions of "two R₂ at adjacent ring atoms form a C₃₋₄alkylene group, wherein 1-2 carbon atoms may be replaced by X₂" or "two R₅ at adjacent ring atoms form a C₃₋₄alkylene group, wherein 1-2 carbon atoms may be replaced by X₃" encompass -CH₂-CH₂-CH₂-, -CH₂-CH₂-CH₂-CH₂-, -O-CH₂-O-, -O-CH₂-CH₂-O- and -CH₂-CH₂-NH-. An example of a substituted group is -CH₂-CH₂-N(CH₃)-,

In the context of the invention, the definition of A₁ or A₃ as a "five- to ten-membered monocyclic or fused polycyclic aromatic ring system" encompasses a C₆- or C₁₀-aromatic hydrocarbon group or a five- to ten-membered heterocyclic aromatic ring system. "Polycyclic" means preferably bicyclic.

In the context of the invention, the definition of R₂ as a "three- to six-membered monocyclic ring system" encompasses a C₆-aromatic hydrocarbon group, a five- to six-membered heterocyclic aromatic ring system and a three- to six-membered monocyclic aliphatic or heterocyclic ring system.

A C₆- or C₁₀-aromatic hydrocarbon group is typically phenyl or naphthyl, especially phenyl.

Preferably, but also depending on substituent definition, "five- to ten-membered heterocyclic aromatic ring systems" consist of 5 to 10 ring atoms of which 1-3 ring atoms are hetero atoms. Such heterocyclic aromatic ring systems may be present as a single ring system or as bicyclic or tricyclic ring systems; preferably as single ring systems or as benz-annelated ring systems. Bicyclic or tricyclic ring systems may be formed by annelation of two or more rings, or by a bridging atom, e.g. oxygen, sulfur, nitrogen. Examples of heterocyclic ring systems are: imidazo[2,1-b]thiazole, pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazolidine, isothiazole, isothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pteridine, and the corresponding benzannelated heterocycles, e.g. indole, isoindole, coumarin, isoquinoline, quinoline and the like. Preferred heterocycles are: imidazo[2,1-b]thiazole, oxazole, isoxazole, thiazole, isothiazole, triazole, pyrrole, furane, tetrahydrofurane, pyridine, pyrimidine, imidazole or pyrazole.

In the context of the invention, three- to six-membered monocyclic aliphatic ring systems are typically cyclopropyl, cyclobutyl, cyclopentyl or cyclohexyl.

The acid addition salts of compounds of formula (I) or of compounds of formula (II) are pharmaceutically acceptable salts, Such salts are known in the field (e.g. S.M. Berge, et al, "Pharmaceutical Salts", J. Pharm. Sd., 1977, 66:1-19; and "Handbook of Pharmaceutical Salts, Properties, Selection, and Use", Stahl, RH., Wermuth, C.G., Eds.; Wiley-VCH and VHCA: Zurich, 2002), A "pharmaceutically acceptable salt" is intended to mean a salt of a free base of a compound represented by formula (I) or by formula (II) that is not toxic, biologically intolerable, or otherwise biologically undesirable. Preferred pharmaceutically acceptable salts are those that are pharmacologically effective and suitable for contact with the tissues of patients without undue toxicity, irritation, or allergic response.

The compounds of formula (I) or compounds of formula (II) and their manufacture are known from WO2001/85727, WO2004/022556, WO2005/118535, WO2005/123732, WO2006/008608, WO2007/045478, WO2007/068476 and WO2007/068475, or can be prepared analogously to said references. WO2001/85727, WO2004/022556, WO2005/118535, WO2005/123732, WO2006/005608, WO2007/045478, WO2007/068476 and WO2007/068475 are incorporated herein by reference.

On account of asymmetrical carbon atom(s) that may be present in the compounds of formula (I) and compounds of formula (II), the compounds may exist in optically active form or in form of mixtures of optical isomers, e.g. in form of racemic mixtures or diastereomeric mixtures. All optical isomers and their mixtures, including racemic mixtures, are part of the present invention.

In one embodiment of the invention, a compound of formula (I) is used.

In one embodiment of the invention, a compound of formula (I) is used 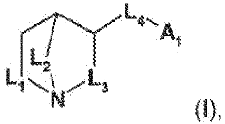 wherein
L₁ is -CH₂-; L₂ is -CH₂-CH₂-, and L₃ is -CH₂- or -CH(CH₃)-,
L₄ is a group selected from 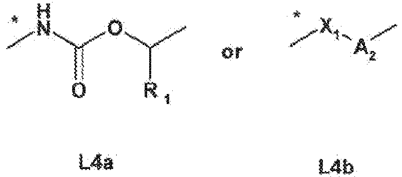 wherein the bond marked with the asterisk is attached to the azabicycloalkyl moiety;
R₁ is hydrogen or C₁₋₄alkyl;
X₁ is -O- or -NH-;
A₂ is selected from 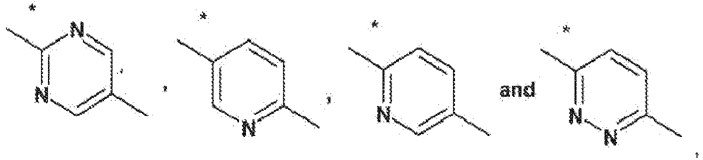 wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and each R₂ independently is C₁₋₆alkyl, C₁₋₆halogenalkyl, C₁₋₆alkoxy, C₁₋₆halogenalkoxy or halogen.

In one embodiment of the invention, a compound of formula (I) is used 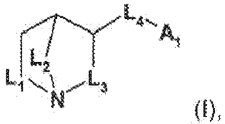 wherein
L₁ is -CH₂-; L₂ is -CH₂-CH₂-; and L₃ is -CH₂-;
L₄ is 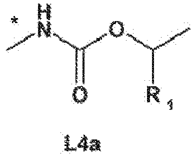 wherein the bond marked with the asterisk is attached to the azabicycloalkyl moiety;
R₁ is hydrogen or C₁-₄alkyl;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and each R₂ independently is C₁₋₆alkyl, C₁₋₆halogenalkyl, C₁₋₆alkoxy, C₁₋₆halogenalkoxy or halogen.

In one embodiment of the invention, a compound of formula (I) is used 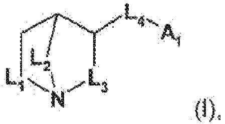 wherein
L₁ is -CH₂-; L₂ is -CH₂-CH₂-; and L₃ is -CH₂- or -CH(CH₃)-;
L₄ is 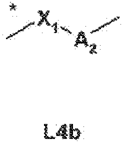 wherein the bond marked with the asterisk is attached to the azabicycloalkyl moiety;
X₁ is -O- or -NH-;
A₂ is selected from 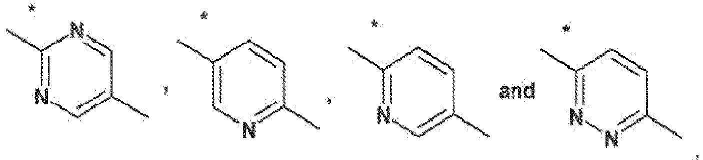 wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen; and each R₂ independently is C₁₋₆alkyl, C₁₋₆halogenalkyl, C₁-₆alkoxy, C₁₋₆halogenalkoxy or halogen.

In one embodiment of the invention, a compound of formula (I) is used 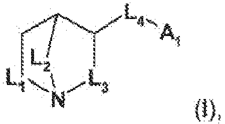 wherein
L₁ is -CH₂-CH₂-, L₂ is -CH₂-; and L₃ is -CH₂-CH₂-;
L₄ is 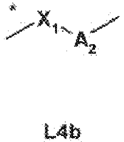 wherein the bond marked with the asterisk is attached to the azabicycloalkyl moiety;
X₁ is -O- or -NH-;
A₂ is selected from 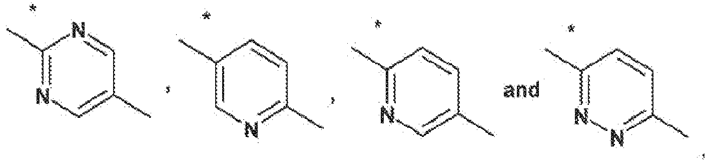 wherein the bond marked with the asterisk is attached to X₁;
A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur, atoms, and wherein the ring system may be substituted once or more than once by R₂, and wherein a substituent on a nitrogen in a heterocyclic ring system may not be halogen and each R₂ independently is C₁₋₆alkyl, C₁₋₆halogenalkyl, C₁₋₆alkoxy, C₁₋₆halogenalkoxy or halogen.

In one embodiment of the invention, a compound of formula (II) is used.

In one embodiment of the invention, a compound of formula (II) is used 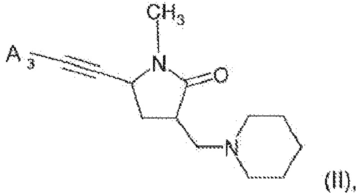 wherein
A₃ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₅, and wherein a substituent on a nitrogen in a heterocyclic ring system may not be halogen; and each R₅ independently is C₁₋₆alkyl, C₁₋₆halogenalkyl, C₁₋₆alkoxy, C₁₋₆halogenalkoxy, amino or halogen.

In one embodiment of the invention, the compound of formula (I) is a compound selected from the group consisting of
A-1: (S)-(1-aza-bicyclo[2.2.2]oct-3-yl)-carbamic acid (S)-1-(2-fluoro-phenyl)-ethyl ester;
A-2: (S)-(1-aza-bicyclo[2.2.2]oct-3-yl)-carbamic acid (R)-1-(2-chtoro-phenyl)-ethyl ester;
A-3: (S)-(1-aza-bicyclo[2.2.2]oct-3-yl)-carbamic acid (S)-1-phenyl-ethyl ester;
B-1: (R)-3-(6-phenyl-pyrimidin-2-yloxy)-1-aza-bicyclo[2.2.2]octane;
B-2: (R)-3-(5-p-tolyl-pyrimidin-2-yloxy)-1-aza-bicyclo[2.2.2]octane;
B-3: (R)-3-(5-(2-fluoro-4-methyl-phenyl)-pyrimidin-2-yloxy)-1-aza-bicyclo[2.2.2]octane;
B-4: (R)-3-(5-(3,4-dimethyl-phenyl)-pyrimidin-2-yloxy)-1-aza-bicyclo[2.2.2]octane;
B-5: (R)-3-(6-p-tolyl-pyridin-3-yloxy)-1-aza-biryclo[2.2,2]octane;
B-6: (R)-3-(6-phenyl-pyridin-3-yloxy)-1-aza-bicyclo[2.2.2]octane;
B-7: (R)-3-(6-(3,4-dimethyl-phenyl)-pyridin-3-yloxy)-1-aza-bicyclo[2.2.2]octane;
B-8: (R)-3-[6-(2-fluoro-4-methyl-phenyl)-pyridazin-3-yloxy]-1-aza-bicyclo[2,2.2]octane;
B-9: (R)-3-[6-(4,5-dimethyl-2-fluoro-phenyl)-pyridazin-3-yloxy]-1-aza-bicyclo[2.2.2]octane;
B-10: (R)-3-[6-(3,4-dimethyl-phenyl)-pyridazin-3-yloxy]-1-aza-bicyclo[2.2.2]octane;
B-11: (R)-3-[6-(4-methyl-phenyl)-pyridazin-3-yloxy]-1-aza-bicyclo[2.2.2]octane;
B-12: (R)-3-[6-(2,5-difluoro-4-methyl-phenyl)-pyridazin-3-yloxy]-1-aza-bicyclo[2.2.2]octane;
B-13: (2S,3R)-3-[6-(1H-indol-5-yl)-pyridazin-3-yloxy]-2-methyl-1-aza-bicyclo[2.2.2]octane;
B-14: (2R,3S)-3-[6-(1H-indol-5-yl)-pyridazin-3-yloxy]-2-methyl-1-aza-bicyclo[2.2.2]octane;
B-15: (2S,3R)-3-[5-(1H-indol-5-yl)-pyrimidin-3-yloxy]-2-methyl-1-aza-bicyclo[2.2.2]octane;
B-16: (2R,3S)-3-[5-(1H-indol-5-yl)-pyrimidin-2-yloxy]-2-methyl-1-aza-bicyclo[2.2.2]octane;
B-17: 3-[6-(1H-indol-5-yl)-pyrdin-3-yloxy]-2-methyl-1-aza-bicyclo[2.2.2]octane;
B-18: (2S,3,R)-2-methyl-3-[6-(5-methyl-thiophen-2-yl)-pyridazin-3-yloxy]-1-aza-bicyclo[2.2.2]octane;
B-19: 3-[8-(2,3-dimethyl-1H-indol-5-yl)-pyridazin-3-yloxy]-2-methyl-1-aza-bicyclo[2-2.2]octane;
B-20: trans-2-methyl-1-aza-bicyclo[2.2.2]oct-3-yl)-(6-phenyl-pyridin-3-yl)-amine;
B-21: trans -[6-(1H-indol-5-yl)-pyridin-3-yl]-(2-methyl-1-aza-bicyclo[2,2.2]oct-3-yl)-amine;
C-1: (4S,5R)-4-[5-(1H-indol-5-yl)-pyrimidin-2-yloxy]-1-aza-bicyclo[3.3.1]nonane;
C-2: 5-(2-[(4S,5R)-(1-aza-bicyclo[3.3.1]non-4-yl)oxy]-pyrimidin-5-yl)-1,3-dihydro-indol-2-one;
C-3: (4S,5R)-4-.[6-(1H-indol-5-yl)-pyridin-3-yloxy]-1-aza-bicyclo[3.3.1]nonane;
C-4: (4S,5R)-4-[5-(1H-indol-5-yl)-pyridin-2-yloxy]-1-aza-bicyclo[3.3.1]nonane;
C-5: (4S,5R)-4-[6-(1H-indol-5-yl)-pyridazin-3-ylxy]-1-aza-bicyclo[3,3.1]nonane;
C-6: 5-{6-[(4S,5R)-(1-aza-bicyclo[3.3.1]non-4-yl)oxy]-pyridazin-3-yl}-1,3-dihydro-indol-2-one;
C-7: (1-aza-bicyclo[3.3.1]non-4-yl)-[5-(1H-indol-5-yl)-pyridin-2-yl]-amine;
C-8: (1-aza-bicyclo[3.3.1]non-4-yl)-[5-(1H-indol-5-yl)-pyrimidin-2-yl]-amine;
C-9: (1-aza-bicyclo[3.3.1]non-4-yl)-[6-(1H-indol-5-yl)-pyridin-3-yl]-amine;
C-10: (1-aza-bicyclo[3.3.1]non-4-yl)-[6-(1H-indol-5-yl)-pyridin-3-yl]-amine;
C-11: (1-aza-bicyclo[3.3.1]non-4-yl)-[5-(1H-indol-4-yl)-pyrimidin-2-yl]-amine;
C-12: (1-aza-bicyclo[3.3.1]non-4-yl)-[6-(1H-indol-5-yl)-pyridazin-3-yl]-amine;
D-1: 5-benxofuran-5-ylethynyl-1-methyl-3-piperidin-1-ylmethyl-pyrrolidin-2-one;
D-2: 1-methyl-5-phenylethynyl-3-piperidin-1-ylmethyl-pyrrolidin-2-one;
D-3: 1-methyl-5-(1-methyt-1H-indol-5-ylethynyl)-3-piperidin-1-ylmethyl-pyrrolidin-2-one; and
D-4: 5-(3-Amino-phenylethynyl)-1-methyl-3-piperidin-1-ylmethyl-pyrrolidin-2-one.

In one embodiment of the invention, the compound of formula (I) is a compound selected from the group consisting of compound A-1, A-2 and A-3.

In one embodiment of the invention, the compound of formula (I) is a compound selected from the group consisting of compound B-1, B-2, B-3, B-4, B-5, B-6, B-7, B-8, B-9, B-10, B-11, B-12, B-13, B-14, 8-15, B-16, B-17, B-18, B-19, B-20 and B-21.

In one embodiment of the invention, the compound of formula (I) is a compound selected from the group consisting of compound C-1, C-2, C-3, C-4, C-5, C-6, C-7, C-8, C-9, C-10, C-11 and C-12.

In one embodiment of the invention, the compound of formula (II) is a compound selected from the group consisting of compound D-1, D-2, D-3 and D-4.

In one embodiment, the compounds of formula (I) or compounds of formula (II) are for the treatment of ataxia.

In one embodiment, the compounds of formula (I) or compounds of formula (II) are for the prevention of ataxia.

In one embodiment, the compounds of formula (I) or compounds of formula (II) are for the delay of ataxia.

Treatment may comprise a reduction in the symptoms associated with ataxia, including for example, although not limited to, an improvement of gait, balance, limb coordination and/or speech; or an increased period of time between episodes of ataxia,

In the case of prophylactic treatment, the compounds of formula (I) or componds of formula (II) may be used to delay or prevent the onset of ataxia.

In one embodiment, the compounds of formula (I) or compounds of formula (II) are for the treatment, prevention or delay of hereditary ataxia.

In one embodiment, the compounds of formula (I) or compounds of formula (II) are for the treatment, prevention or delay of autosomal recessive ataxia.

In one embodiment, the compounds of formula (I) or compounds of formula (II) are for the treatment, prevention or delay of Friedreichs ataxia.

In one embodiment, the compounds of formula (I) or compounds of formula (II) are for the treatment, prevention or delay of Spinocerebellar ataxia.

In one embodiment, the compounds of formula (I) or compounds of formula (II) are for the treatment, prevention or delay of Spinocerebellar ataxia types 1, 2, 3, 6, 7 or 17, especially Spinocerebellar ataxia type 3 (Machado-Joseph disease).

In one embodiment, the compounds of formula (I) or compounds of formula (II) are for the treatment, prevention or delay of Spinocerebellar ataxia types 8, 10 or 12.

In one embodiment, the compounds of formula (I) or compounds of formula (II) are for the treatment, prevention or delay of Spinocerebellar ataxia types 5, 13, 14 or 27, especially Spinocerebellar ataxia type 14.

For the above-mentioned indications (the conditions and disorders) the appropriate dosage will vary depending upon, for example, the compound employed, the host, the mode of administration and the nature and severity of the condition being treated. However, in general, satisfactory results in animals are indicated to be obtained at a daily dosage of from about 0.01 to about 100 mg/kg body weight, preferably from about 0.1 to about 10 mg/kg body weight, e.g 1 mg/kg. In larger mammals, for example humans, an indicated daily dosage is in the range from about 0.1 to about 1000 mg, preferably from about 1 to about 400 mg, most preferably from about 10 to about 100 mg of a compound of formula (I) or a compound of formula (II) conveniently administered, for example, in divided doses up to four times a day.

For use according to the invention, the compounds of formula (I) or compounds of formula (II) may be administered as single active agent or in combination with other active agents, in any usual manner, e.g. orally, for example in the form of tablets or capsules, parenterally, for example in the form of injection solutions or suspensions, or transdermally, for example in the form of a patch.

In one embodiment, the manner of administration is oral administration, for example in the form of tablets or capsules.

In one embodiment, the manner of administration is transdermal administration, for example in the form of a patch.

Moreover, the present invention provides a pharmaceutical composition comprising a a compound of formula (I) or a compound of formula (II) in association with at least one pharmaceutical carrier or diluent for the treatment, prevention or delay of progression of ataxia. Such compositions may be manufactured in conventional manner. Unit dosage forms may contain, for example, from about 2.5 to about 25 mg of one or more of the compounds of formula (I) or compounds of formula (II).

The pharmaceutical compositions according to the invention are compositions for enteral administration, such as rectal or oral administration; or parenteral administration, such as intravenous, nasal or transdermal (e.g. by patch) administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known per se, for example by means of conventional dissolving, lyophilizing, mixing, granulating or confectioning processes. Such processes are exemplified in WO 2005/079802, WO 2003/047581, WO 2004/000316, WO 2005/044265, WO 2005/044266, WO 2005/044267, WO 2006/114262 and WO 2007/071358.

Compositions for transdermal are described in Remington's Pharmaceutical Sciences 16th Edition Mack; Sucker, Fuchs and Spieser, Pharmazeutische Technologie, 1st Edition, Springer.

The usefulness of the compounds of formula (I) or compounds of formula (II) in the treatment of the above-mentioned disorders can be confirmed in a range of standard tests including those indicated below.

### 1. in-vitro Tests

### 1.1. Activity at α7-nAChR, Selectivity against α4β2-nAChR

Based on the activity/selectivity data shown below it is concluded that said compounds are selective agonists at the α7∼nAChR.

| Compound | α7-nAChR activity | | α4β2-nAChR activity | | |
|---|---|---|---|---|---|
| | Potency EC₅₀ (nM) | Efficacy compared to epibatidine (100%) | IC₅₀ (nM) | EC₅₀ (nM) | fold selectivity |
| A-1 | 100 | 83 | 23442 | >100 000 | 234 |
| C-1 | 24 | 84 | 9333 | >100 000 | 388 |
| B-13 | 13 | 89 | 4217 | >100 000 | 324 |

Assays: To assess α7∼nAChR activity, a functional assay was employed using GH3 cells that recombinantly expressed human α7-nAChR. 5 x 104 cells per well were seeded 72 h prior to the experiment on black 96-well plates (Costar) and incubated at 37°C in a humidified atmosphere (5 % CO₂/95 % air). On the day of the experiment, medium was removed by flicking the plates and replaced with 100 µl growth medium containing 2 mM Fluo-4, (Molecular Probes) in the presence of 2.5 mM probenecid (Sigma). The cells were incubated at 37oC in a humidified atmosphere (5 % CO2/95 % air) for 1 h. Plates were flicked to remove excess of Fluo∼4, washed twice with Hepes-buffered salt solution (in mM: NaCl 130, KCl 5.4, CaCl2 2, MgSO4 0.8, NaH2PO4 0.9, glucose 25, Hepes 20, pH 7.4; HBS) and refilled with 100 µl of HBS containing antagonist when appropriate. The incubation in the presence of the antagonist lasted 3-5 minutes. Plates were placed in the cell plate stage of a FLIPR device (fluorescent imaging plate reader, Molecular Devices, Sunnyvale, CA, USA). After recording of the baseline (leaser: excitation 488 nm at 1 W, CCD camera opening of 0.4 seconds) the agonists (50 µl) were added to the cell plate using the FLIPR 96-tip pipettor while simultaneously recording the fluorescence. Calcium kinetic data were normalized to the maximal fitted response induced by epibatidine, which is a full agonist at α7-nAChR. Four parameter Hill equations were fitted to the concentration-response. Values of Emax (maximal effect in % compared to the epibatidine response) and EC50 (concentration producing half the maximal effect in µM) were derived from this fit.

Assay described in: D Feuerbach et al, Neuropharmacology (2005), 48, 215-227.

To assess the activity of the compound of the invention on the human neuronal nAChR α4β2, a similar functional assay is carried out using a human epithelial cell line stably expressing the human α4β2 subtype (Michelmore et al., Naunyn-Schmiedeberg's Arch. Pharmacol. (2002) 366, 235).

### 2. In-vivo Preclinical Tests

### 2.1. Oral bioavailability and brain penetration in Mice

Based on the pharmacokinetic data shown below it is concluded that the brain concentration of said compounds in mice is beyond (or at least equal) to the compounds EC₅₀ at the α7-nAChR for at least 4 hours following an acute oral dose of 30 µmol/kg,

### Compound A-1:

| Administration | Time (hour) | Plasma (pmoles/ml±SD) | Brain (pmoles/g±SD) | Ratio Brain/plasma |
|---|---|---|---|---|
| 30 µmol/kg p.o. | 0.5 | 634.9 ± 261.3 | 706.3 ± 153.4 | 1.1 |
| 30 µmol/kg p.o. | 1 | 684.7 ± 339.6 | 573.7 ± 109.3 | 0.8 |
| 30 µmol/kg p.o. | 2 | 168.2 ± 91.3 | 191.9 ± 34.9 | 1.1 |
| 30 µmol/kg p.o. | 4 | 85.0 ± 54.3 | 104.6 ± 39.6 | 1.2 |
| 30 µmol/kg p.o. | 6 | 29.5 ± 13.8 | 40.5 ± 12.1 | 1.4 |
| 30 µmol/kg p.o. | 24 | 3.8 ± 0.6 | 9.1 ± 2.7 | 2.4 |

### Compound B-13:

| Administration | Time (hour) | Plasma (pmoles/ml±SD) | Brain (pmoles/g±SD) | Ratio Brain/plasma |
|---|---|---|---|---|
| 30 µmol/kg p.o. | 0.25 | 2196 ± 397 | 1884 ± 291 | 0.86 |
| 30 µmol/kg p.o. | 0.5 | 2265 ± 419 | 2960 ± 706 | 1.31 |
| 30 µmol/kg p.o. | 1 | 1554 ± 523 | 2940 ± 335 | 1.89 |
| 30 µmol/kg p.o. | 2 | 1172 ± 252 | 1260 ± 172 | 1.07 |
| 30 µmol/kg p.o. | 4 | 429 ± 167 | 379 ± 134 | 0.88 |
| 30 µmol/kg p.o. | 8 | 80 ± 23 | 93 ± 30 | 1.17 |
| 30 µmol/kg p.o. | 24 | * | 13 ± 4 | |

### Compound C-1:

| Administration | Time (hour) | Plasma (pmoles/ml±SD) | Brain (pmoles/g±SD) | Ratio Brain/plasma |
|---|---|---|---|---|
| 30 µmol/kg p.o. | 0.25 | 1601 ± 758 | 620 ± 221 | 0.39 |
| 30 µmol/kg p.o. | 0.5 | 3414 ± 956 | 1405 ± 539 | 0.41 |
| 30 µmol/kg p.o. | 1 | 1241 ± 583 | 1458 ± 189 | 1.17 |
| 30 µmol/kg p.o. | 2 | 875 ± 261 | 1478 ± 259 | 1 69 |
| 30 µmol/kg p.o. | 4 | 762 ± 159 | 842 ± 187 | 1.11 |
| 30 µmol/kg p.o. | 8 | 239 ± 27 | 362 ± 62 | 1.51 |
| 30 µmol/kg p.o. | 24 | * | * | |

Assay: Compounds were orally (30 µmol/kg) administered. Male mice (30-35g, OF1/ICstrain) were sacrificed at indicated time points after oral administration. Trunk- blood was collected in EDTA-containing tubes and the brain was removed and immediately frozen on dry ice.

To 100 µl plasma 10 µl internal standard (1.0 pmol of a compound with solubility and ionization properties similar to test compounds) was added and extracted three times with 500 µl dichloromethane. The combined extracts were then dried under a stream of nitrogen and re-dissolved in 100 µl acetonitrile/water (70% acetonitrile). Brains were weighed and homogenized in water (1:5 w/v). Two 100 µl aliquots of each homogenate + 10 µl of internal standard (same standard as used for the plasma samples) were extracted three times with 500 µl dichloromethane and further processed as the plasma samples. Samples were separated on Beckmann high-performance liquid chromatography equipment system with an autosampler (Gilson 233XL). A 10 min linear gradient (10- 70%) of acetonitrile containing 0.5 % (v/v) formic acid was used to elute the compounds from Nucleosil CC-125/2 C18 reversed phase (Machery&Nagel) column.

The limit of detection (LOD), defined as the lowest concentration of the extracted standard sample with a signal to noise ratio of - 3.

### 2.2. Functional read-out in Mice (Social Recognition Test)

Based on the functional in-vivo data shown below it is concluded that oral dosing of said compounds at relevant concentrations lead to a specific effect associated with α7-nAChR (i.e. cognition enhancement in the Social Recognition Test in mouse).

| Compound | Reduction in time scrutinizing in % ± SEM at 24 h | Dose in mg/kg |
|---|---|---|
| A-1 | 52 ± 4 | 3 |
| C∼1 | 51 ± 3 | 0.3 |
| B∼13 | 37 ± 7 | 0.3 |

Assay: Social interactions between two experimental animals are influenced by their degree of familiarity: the better they know each other, the less time they spend on mutual scrutiny at each meeting. In agreement with published data in rats (Mondadon et al., 1993) we have observed (i) that an adult mouse shows a shortened scrutiny of a young conspecific if the two mice are brought together again within a short time interval (e.g. 1 hour), (ii) that this curtailment is attributable to memory processes: it does not occur if the familiar young partner is replaced by a strange (unfamiliar) young mouse on the second occasion and (iii) that the adult mouse's recollection of the previously scrutinized juvenile partner fades with the elapsed time, i.e., after 24 h, scrutiny takes just about as long as at the first encounter. Memory enhancing agents (i.e. oxiracetam) facilitate learning to the extent that the previously met (familiar) partner is still remembered after 24 h, whereas in vehicle treated control animals the memory usually fades after less than 1 hour (Thor and Holloway, 1982) or after 2-3 hours.

Baseline-test: Pairs consisting of one adult and one young mouse were assigned at random to the experimental and control groups. In each pair only the adult mouse was orally treated 1 hour before the trial with either vehicle or the test compound. The duration of active contacts of the adult mouse with the young mouse was manually recorded over a period of 3 min, including the following behavioural, approach-related items: sniffing, nosing, grooming, licking, pawing and playing, anogenital exploration and orientation toward the young mouse; orientation, thereby, was defined as tip of nose of the adult mouse less than approximately 1cm distant from the young mouse's body.

Re-test: Twenty-four hours after the baseline-test, the adults in each treatment group were confronted again with the previously encountered (familiar) partner, whereas the half of the adult animals were put together with the previously encountered (familiar) partner and the other half with another (unfamiliar) young mouse. Again the duration of active approach-behaviours was recorded during a 3-min period. Prior to re-test no oral injection was given. In the table the reduction in time scrutinizing the familiar partner at time 24 compared with the familiar partner at time 0 minutes is given (value of zero would signify no reduction).

### 2.3. Assessment of anti-ataxic effect in animal models

Method: "Purkinje cell degeneration" mice (pcd mice) are considered a valid mouse model of cerebellar ataxia. Pcd mice bear a mutation in the Nna1 gene, a putative zinc protease induced by axotomy (A Fernandez-Gonzalez et al, Science, 295: 1904-1906, 2002), These mice also show delayed degeneration of other brain areas, with retinal photoreceptor degeneration starting a 1 year of age. Concomitant Purkinje cell death and photoreceptor degeneration is also found in Spino-Cerebellar Ataxia-7 (SCA7).

Assessment: Motor performance is quantified by the ability to remain in position on a rotating rod ("rota-rod" test, see: AM Fernandez et al, Proc Natl Acad Sci USA, 95: 1253-1258, 1998). Mice obtaining vehicle controls and acute/repeated dosing of compounds of formula (I) or compounds of formula (II) are analyzed.

Protocol: Mice are tested at different ages. One group of mice is treated with an effective dose of a compound of formula (I) or a compound of formula (II), the control group receives the vehicle only. The compounds of formula (I) or compounds of formula (II) are tested for motor coordination on the rotating rod acutely and after repeated dosing.

### 3. Clinical Testing Improvement Trials

Clinical testing of the compounds of formula (I) or compounds of formula (II) may be conducted, for example, in one of the following study designs. The skilled physician may look at a number of aspects of behaviors and abilities of patients. He will realize that such studies are considered as guidelines and the certain aspects of the studies may be modified and redefined depending on the circumstance and environment, for example.

### 3.1 Trial A: Normal Patient Population

A patient population, with a normal control is dosed once a day for a week or longer tested. The test is designed to allow for improvement, i.e. that there is a measurable parameter increase of the impaired function. The patients are tested at the beginning and at the end of the dosage period and the results are compared and analyzed.

### 3.2 Trial B: Deficit population

A patient population with a deficit associated with ataxia is dosed once a day for a week or longer and tested. The test is designed to allow for improvement, i.e. that there is a measurable parameter increase of the impaired function. The patients are tested at the beginning and at the end of the dosage period and the results are compared and analyzed.

### 3.3 Considerations for designing a trial

- When designing a trial, the skilled person will appreciate the need to protect both against floor and ceiling effects. In other words, the study designing should allow cognition to the measurably raised or lowered.
- Conditions that artificially impair a function, e.g. cognition, are one way to test enhancement of that function. Such conditions are, for example, sleep deprivation and pharmacological challenges.
- Placebo control is required for all trials.
- In assessing the data, evaluation of the likelihood of learning and practice effects from repeat assessments must be made. The likelihood of such effects contaminating the data to produce false positives should be taken in to account when designing the test, e.g. the tests should not be identical (e.g, commit the same list of words to memory) but designed to study the same mechanism. Other countermeasures may include single testing at the end of a trial only.

The invention includes the following aspects:
Aspect 1. A compound selected from the group consisting of
   (A) a compound of formula (I) 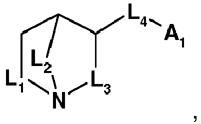 wherein
      L₁ is -CH₂-; L₂ is -CH₂- or -CH₂-CH₂-; and L₃ is -CH₂- or -CH(CH₃)-; or
      L₁ is -CH₂-CH₂-; L₂ is -CH₂-; and L₃ is -CH₂-CH₂-;
      L₄ is a group selected from 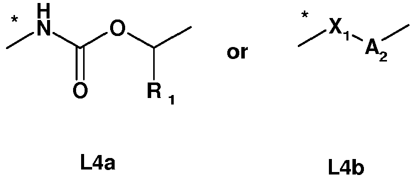 wherein the bond marked with the asterisk is attached to the azabicycloalkyl moiety;
      R₁ is hydrogen or C₁₋₄alkyl;
      X₁ is -O- or -NH-;
      A₂ is selected from 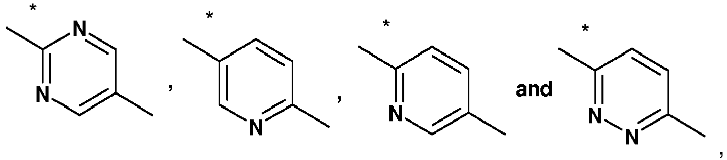 wherein the bond marked with the asterisk is attached to X₁;
      A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen;
      each R₂ independently is C₁₋₆alkyl, C₁₋₆halogenalkyl, C₁₋₆alkoxy, C₁₋₆halogenalkoxy, halogen, cyano or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen;
      or two R₂ at adjacent ring atoms form a C₃₋₄alkylene group, wherein 1-2 carbon atoms may be replaced by X₂, and wherein the C₃₋₄alkylene group may be substituted once or more than once by R₃;
      each X₂ independently is -O- or -N(R₄)-;
      each R₄ independently is hydrogen or C₁₋₆alkyl; and
      each R₃ independently is halogen or C₁₋₆alkyl; and
   (B) a compound of formula (II), 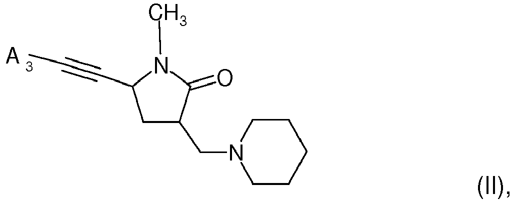 wherein
      A₃ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₅, and wherein a substituent on a nitrogen in a heterocyclic ring system may not be halogen;
      each R₅ independently is C₁₋₆alkyl, C₁₋₆halogenalkyl, C₁₋₆alkoxy, C₁₋₆halogenalkoxy, halogen, cyano, amino or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen;
      or two R₅ at adjacent ring atoms form a C₃₋₄alkylene group, wherein 1-2 carbon atoms may be replaced by X₃, and wherein the C₃₋₄alkylene group may be substituted once or more than once by R₆;
      each X₃ independently is -O- or -N(R₇)-;
      each R₇ independently is hydrogen or C₁₋₆alkyl; and
      each R₆ independently is halogen or C₁₋₆alkyl;
      in free base form or in pharmaceutically acceptable acid addition salt form for use in the treatment, prevention or delay of progression of ataxia.
Aspect 2. A compound of formula (I) 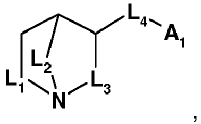 wherein
   L₁ is -CH₂-; L₂ is -CH₂- or -CH₂-CH₂-; and L₃ is -CH₂- or -CH(CH₃)-; or
   L₁ is -CH₂-CH₂-; L₂ is -CH₂-; and L₃ is -CH₂-CH₂-;
   L₄ is a group selected from 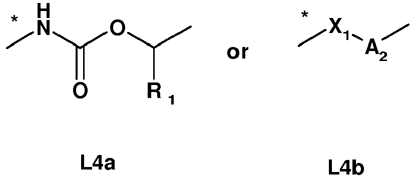 wherein the bond marked with the asterisk is attached to the azabicycloalkyl moiety;
   R₁ is hydrogen or C₁₋₄alkyl;
   X₁ is -O- or -NH-;
   A₂ is selected from 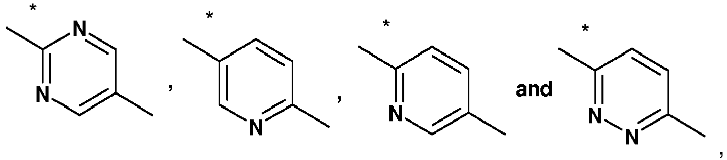 wherein the bond marked with the asterisk is attached to X₁;
   A₁ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₂, and wherein a substituent on a nitrogen in a heterocyclic ring system may not be halogen;
   each R₂ independently is C₁₋₆alkyl, C₁₋₆halogenalkyl, C₁₋₆alkoxy, C₁₋₆halogenalkoxy, halogen, cyano or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen;
   or two R₂ at adjacent ring atoms form a C₃₋₄alkylene group, wherein 1-2 carbon atoms may be replaced by X₂, and wherein the C₃₋₄alkylene group may be substituted once or more than once by R₃;
   each X₂ independently is -O- or -N(R₄)-;
   each R₄ independently is hydrogen or C₁₋₆alkyl; and
   each R₃ independently is halogen or C₁₋₆alkyl;
   in free base form or in pharmaceutically acceptable acid addition salt form for use in the treatment, prevention or delay of progression of ataxia.
Aspect 3. A compound of formula (II) 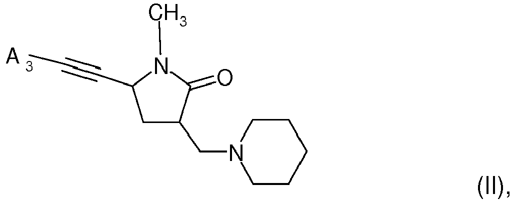 wherein
   A₃ is a five- to ten-membered monocyclic or fused polycyclic aromatic ring system which may contain from 1 to 4 hetero atoms selected from nitrogen, oxygen and sulfur, wherein the ring system may contain not more than 2 oxygen atoms and not more than 2 sulfur atoms, and wherein the ring system may be substituted once or more than once by R₅, and wherein a substituent on a nitrogen in a heterocyclic ring system may not be halogen;
   each R₅ independently is C₁₋₆alkyl, C₁₋₆halogenalkyl, C₁₋₆alkoxy, C₁₋₆halogenalkoxy, halogen, cyano, amino or a three- to six-membered monocyclic ring system which may be aromatic, saturated or partially saturated and which may contain from 1 to 4 hetero atoms selected from nitrogen, oxygen and sulfur, and wherein each ring system may contain not more than 2 oxygen atoms and not more than 2 sulfur atoms, and wherein each ring system may in turn be substituted once or more than once by C₁₋₆alkyl, C₁₋₆halogenalkyl, C₁₋₆alkoxy, C₁₋₆halogenalkoxy, halogen or cyano, and wherein a substituent on a nitrogen in a heterocyclic ring system may not be halogen;
   or two R₅ at adjacent ring atoms form a C₃₋₄alkylene group, wherein 1-2 carbon atoms may be replaced by X₃, and wherein the C₃₋₄alkylene group may be substituted once or more than once by R₆;
   each X₃ independently is -O- or -N(R₇)-;
   each R₇ independently is hydrogen or C₁₋₆alkyl; and
   each R₆ independently is halogen or C₁₋₆alkyl;
   in free base form or in pharmaceutically acceptable acid addition salt form for use in the treatment, prevention or delay of progression of ataxia.
Aspect 4. A method for the treatment, prevention or delay of progression of ataxia in a subject in need of such treatment, which comprises administering to said subject a therapeutically effective amount of a compound of formula (I) as defined in aspect 1 or of a compound of formula (II) as defined in aspect 1.
Aspect 5. A pharmaceutical composition comprising a compound of formula (I) as defined in aspect 1 or of a compound of formula (II) as defined in aspect 1; for the treatment, prevention or delay of progression of ataxia.

## Claims

1. A compound selected from the group consisting of
(R)-3-(6-p-tolyl-pyridin-3-yloxy)-1-aza-bicyclo[2.2.2]octane;
(2S,3R)-3-[6-(1H-indol-5-yl)-pyridazin-3-yloxy]-2-methyl-1-aza-bicyclo[2.2.2]octane;
(4S,5R)-4-[5-(1H-indol-5-yl)-pyrimidin-2-yloxy]-1-aza-bicyclo[3.3.1]nonane;
(S)-(1-aza-bicyclo[2.2.2]oct-3-yl)-carbamic acid (S)-1-(2-fluroro-phenyl)-ethyl ester;
in free base form or in a pharmaceutically acceptable acid addition salt form for use in the treatment, prevention or delay of progression of ataxia.

2. A compound according to claim 1, wherein said compound is
(R)-3-(6-p-tolyl-pyridin-3-yloxy)-1-aza-bicyclo[2.2.2]octane;
in free base form or in pharmaceutically acceptable acid addition salt form for use in the treatment, prevention or delay of progression of ataxia.

3. A compound according to claim 1, wherein said compound is
(S)-(1-aza-bicyclo[2.2.2]oct-3-yl)-carbamic acid (S)-1-(2-fluroro-phenyl)-ethyl ester;
in free base form or in pharmaceutically acceptable acid addition salt form for use in the treatment, prevention or delay of progression of ataxia.

4. A compound according to claim 1, wherein said compound is
(2S,3R)-3-[6-(1H-indol-5-yl)-pyridazin-3-yloxy]-2-methyl-1-aza-bicyclo[2.2.2]octane;
in free base form or in pharmaceutically acceptable acid addition salt form for use in the treatment, prevention or delay of progression of ataxia.

5. A compound according to claim 1, wherein said compound is
(4S,5R)-4-[5-(1H-indol-5-yl)-pyrimidin-2-yloxy]-1-aza-bicyclo[3.3.1]nonane;
in free base form or in pharmaceutically acceptable acid addition salt form for use in the treatment, prevention or delay of progression of ataxia.

6. A compound according to any of claims 1 to 5 for use in the treatment, prevention or delay of hereditary ataxia.

7. A compound according to any of claims 1 to 5 for use in the treatment, prevention or delay of autosomal recessive ataxia.

8. A compound according to any of claims 1 to 5 for use in the treatment, prevention or delay of Friedreichs ataxia.

9. A compound according to any of claims 1 to 5 for use in the treatment, prevention or delay of Spinocerebellar ataxia.

10. A compound according to any of claims 1 to 5 for use in the treatment, prevention or delay of Spinocerebellar ataxia type 1, 2, 3, 6, 7 or 17.

11. A compound according to any of claims 1 to 5 for use in the treatment, prevention or delay of Spinocerebellar ataxia type 8, 10 or 12.

12. A compound according to any of claims 1 to 5 for use in the treatment, prevention or delay of Spinocerebellar ataxia type 5, 13, 14 or 27.
